Europäisches Patentamt

European Patent Office

Office européen des brevets

(19)

(11) Veröffentlichungsnummer: **0 451 746 B1**

## (12) EUROPÄISCHE PATENTSCHRIFT

(45) Veröffentlichungstag der Patentschrift: **19.07.95**

(21) Anmeldenummer: **91105496.3**

(22) Anmeldetag: **08.04.91**

(51) Int. Cl.6: **C07C 17/06**, C07C 19/08,
C07C 17/20, B01J 27/132,
C07C 17/00, B01J 23/16,
B01J 27/08

(54) **Verfahren zur Herstellung von Fluor enthaltenden Ethanderivaten.**

(30) Priorität: **12.04.90 DE 4011789**
**25.02.91 DE 4105832**

(43) Veröffentlichungstag der Anmeldung:
**16.10.91 Patentblatt 91/42**

(45) Bekanntmachung des Hinweises auf die
Patenterteilung:
**19.07.95 Patentblatt 95/29**

(84) Benannte Vertragsstaaten:
**AT BE CH DE DK ES FR GB IT LI NL SE**

(56) Entgegenhaltungen:
EP-A- 0 297 947      EP-A- 0 348 190
EP-A- 0 349 190      WO-A-89/12615
DE-A- 2 234 305      DE-B- 1 245 348
US-A- 3 413 362      US-A- 4 374 289

(73) Patentinhaber: **Kali-Chemie Aktiengesellschaft
Hans-Böckler-Allee 20
D-30173 Hannover (DE)**

(72) Erfinder: **Eicher, Johannes
Moorkamp 7
W-3008 Garbsen 1 (DE)**
Erfinder: **Fazniewscy, Karl-Heinz
Im Gesenk 8
W-3160 Lehrte (DE)**
Erfinder: **Rudolph, Werner
Oderstrasse 38
W-3000 Hannover 71 (DE)**
Erfinder: **Swidersky, Hans-Walter
Zeppelinstrasse 5
W-3000 Hannover 1 (DE)**

(74) Vertreter: **Lauer, Dieter, Dr.
c/o Solvay Deutschland GmbH
Hans-Böckler-Allee 20
D-30002 Hannover (DE)**

**Beschreibung**

Die Erfindung bezieht sich auf ein Verfahren zur Herstellung von Fluor enthaltenden Ethanderivaten der allgemeinen Formel $F_k CH_n Cl_{3-k-n} CZ^1 Z^2 F$, worin k, n, $Z^1$ und $Z^2$ die unten beschriebene Bedeutung besitzen.

Es besteht ein zunehmender Bedarf an umweltverträglichen Halogenkohlenwasserstoffen. Als solche haben sich beispielsweise jene Fluor enthaltenden Ethanderivate erwiesen, welche mindestens ein Wasserstoffatom aufweisen, beispielsweise $CF_3 CH_3$ (R143a), $CF_3 CH_2 Cl$ (R133a) und besonders $CF_3 CHCl_2$ (R123) sowie $CF_3 CH_2 F$ (R134a). Interessant sind aber auch die entsprechenden 1-Fluor- oder 1.1.-Difluorverbindungen, beispielsweise die als umweltverträglich geltenden Verbindungen $CFCl_2 CHCl_2$ oder $CF_2 ClCHCl_2$, die als Kältemittel, Lösungsmittel oder Treibmittel einsetzbar sind.

Industriell werden solche Verbindungen durch katalysierten Halogen-Fluor-Austausch, insbesondere durch Chlor-Fluor-Austausch, aus entsprechend halogenierten Derivaten hergestellt. Die hierbei verwendeten halogenierten Ausgangsverbindungen sind gegenüber einem Halogen-Fluor-Austauch jedoch sehr reaktionsträge. Besonders zur Herstellung höherfluorierter Verbindungen sind drastische Verfahrensbedingungen notwendig. Trotz solcher drastischer Bedingungen, beispielsweise das Arbeiten in der Gasphase, sind die Umsätze gewöhnlich gering. Weiterer Nachteil bekannter Verfahren ist, daß die verwendeten, oft sehr teuren Katalysatoren keine zufriedenstellende Lebensdauer aufweisen.

Die EP-A-0 349 190 offenbart die Herstellung fluorierter Alkane. Dabei werden entsprechende Chlor oder Brom enthaltende Ausgangsverbindungen zwecks Chlor-Fluor-Austausch oder Brom-Fluor-Austausch mit HF in Anwesenheit mindestens eines Katalysators aus der Gruppe umfassend $TaCl_5$ und $TaBr_5$ umgesetzt.

Die US-A 4,374,289 offenbart die Herstellung von 1-Fluor-1,2,2-trichlorethan aus Trichlorethylen und HF in Anwesenheit eines Katalysators ausgewählt aus einer Gruppe verschiedener Katalysatoren, welche u. a. $CF_3 SO_3 H$, $MoCl_5$ und $NbCl_5$ umfaßt.

Aufgabe der vorliegenden Erfindung ist es, ein Verfahren zur Herstellung von Fluor enthaltenden Ethanderivaten zur Verfügung zu stellen, das technisch einfach durchzuführen ist und einen hohen Umsatz aufweist.

Diese Aufgabe wird durch das Verfahren der vorliegenden Erfindung gelöst.

Das erfindungsgemäße Verfahren umfaßt die Herstellung von Fluor enthaltenden Ethanderivaten der allgemeinen Formel (I)

$$F_k H_n Cl_{3-k-n} C\text{-}CZ^1 Z^2 F \qquad (I)$$

worin $Z^1$ und $Z^2$ gleich oder verschieden sein können und Wasserstoff, Fluor, Chlor oder Brom bedeuten und k 0, 1 oder 2 und n 1, 2 oder 3 bedeuten

durch Umsetzung eines halogenierten Alkens oder halogenierten Alkans als Ausgangsverbindung mit Fluorwasserstoff in Anwesenheit eines Katalysatorsystems in flüssiger Phase bei einer Temperatur zwischen 0 und 250 °C, wobei der Fluorwasserstoff mindestens in äquimolarer Menge, bezogen auf die Ausgangsverbindung, in der flüssigen Phase vorhanden ist, das Mol-Verhältnis von Ausgangsverbindung zu Katalysatorsystem zwischen 10:1 bis 1:100 ist, das Katalysatorsystem ein Gemisch aus Metallhalogenid und einem Sulfonsäurederivat im Mol-Verhältnis von Metallhalogenid zu Sulfonsäurederivat zwischen 100:1 und 1:10 umfaßt, wobei das Metallhalogenid ausgewählt ist aus Niobpentahalogenid, Tantalpentahalogenid, Molybdänpentahalogenid oder deren Gemischen und das Sulfonsäurederivat ausgewählt ist aus der Fluorsulfonsäure, Perfluorniedrigalkansulfonsäure mit 1 bis 4 C-Atomen, insbesondere Trifluormethansulfonsäure umfassenden Gruppe und wobei man

a) als halogeniertes Alken eine Verbindung der allgemeinen Formel (II) verwendet

$$F_k H_m Cl_{2-k-m} C = CX^1 X^2 \qquad (II)$$

worin $X^1$ und $X^2$ gleich oder verschieden sein können und Wasserstoff, Fluor, Chlor oder Brom bedeuten, k 0, 1 oder 2 und m 0, 1 oder 2 bedeutet, oder

b) als halogeniertes Alkan eine Verbindung der allgemeinen Formel (III) verwendet

$$F_k H_n Cl_{3-k-n} C\text{-}CY^1 Y^2 Y^3 \qquad (III)$$

worin k und n die obige Bedeutung besitzt, $Y^1$, $Y^2$ gleich oder verschieden sein können und Wasserstoff, Fluor, Chlor oder Brom und $Y^3$ Chlor oder Brom bedeuten.

Im Rahmen der vorliegenden Erfindung ist die Summe aus k und n 1, 2 oder 3 und die Summe aus k und m 0, 1 oder 2.

Bevorzugt steht in den Ausgangsverbindungen der Formel II und III k für 0 und $X^1$, $X^2$, $Y^1$ und $Y^2$ für Fluor, Chlor oder Brom.

Das Verfahren kann bei Drücken zwischen 1 und 100 bar (abs.) und Temperaturen von 0 bis 250 °C betrieben werden. Hierbei werden Druck und Temperatur so gewählt, daß die Umsetzung in der flüssigen Phase abläuft.

Die beim erfindungsgemäßen Verfahren erhaltenen Ethanderivate unterscheiden sich von den Ausgangsverbindadurch, daß sie mindestens ein Fluoratom mehr aufweisen. Für jedes auf diese Weise in das Substratmolekül eingeführte Fluoratom setzt man zweckmäßigerweise Fluorwasserstoff in einer Menge ein, die mindestens der stöchiometrisch notwendigen Menge enspricht. Die zur HF-Anlagerung bzw. zum Halogen-Fluor-Austausch eingesetzte Menge an Fluorwasserstoff kann auch größer sein. Sie kann beispielsweise das bis zu fünfzehnfache und mehr der stöchiometrisch notwendigen Menge betragen.

Bei der Herstellung trifluormethylgruppenhaltiger Derivate aus ungesättigten Verbindungen werden gute Ergebnisse bereits dann erzielt, wenn die eingesetzte Menge an Fluorwasserstoff dem ein- bis zehnfachen der stöchiometrisch notwendigen Menge entspricht.

Die einzusetzende Menge an Fluorwasserstoff kann über diese Menge, die zur Fluorwasserstoffanlagerung und/oder zum Chlor-Fluor-Austausch am Alken oder Alkan benötigt wird, hinausgehen. Wenn man nämlich Metallhalogenide als Katalysatorbestandteil einsetzt, welche Chlorid oder Bromid aufweisen, ist anzunehmen, daß diese Metallhalogenide durch Austausch von Chlor oder Brom gegen Fluor in Form von mehr oder weniger Fluor enthaltenden Metallpentahalogeniden in der Reaktionsmischung vorliegen. So wird Niobpentachlorid möglicherweise in Verbindungen des Typs $NbCl_{5-x}F_x$, Niobpentabromid möglicherweise in Verbindungen des Typs $NbBr_{5-x}F_x$, Tantalpentachlorid in $TaCl_{5-x}F_x$ und Tantalpentabromid in Verbindungen des Typs $TaBr_{5-x}F_x$ umgewandelt. Hierbei bedeutet x eine Zahl zwischen 0 und 5. Entsprechendes gilt für Molybdänhalogenide. Wenn also in einer bevorzugten Ausführungsform als Metallhalogenide Niobpentachlorid bzw. Tantalpentachlorid eingesetzt werden, können diese im Reaktionsgemisch möglicherweise in Form teilfluorierter oder fluorierter Metallverbindungen vorliegen.

Die weiter oben gemachte Angabe zur einzusetzenden Menge an Fluorwasserstoff müßte also vollständig lauten: "Für jedes in das Substratmolekül eingeführte Fluoratom setzt man zweckmäßigerweise Fluorwasserstoff in einer Menge ein, die mindestens der stöchiometrisch notwendigen Menge entspricht, und zusätzlich noch soviel Fluorwasserstoff, wie für einen etwaigen Halogen-Fluor-Austausch des Metallhalogenids benötigt wird." Sinngemäß sind auch die weiter unten gemachten Angaben, die sich mit der Stöchiometrie des für die Fluorwasserstoffaddition und/oder den Halogen-Fluor-Austausch beim Alken oder Alkan einzusetzenden Fluorwasserstoffs befassen, aufzufassen. Der Einfachheit halber wird in diesen Angaben nicht jedesmal ausdrücklich erwähnt, daß zusätzlicher Fluorwasserstoff für den etwaigen Halogen-Fluor-Austausch des Metallhalogenids notwendig sein kann.

Um abzuschätzen, welche Menge zusätzlich an Fluorwasserstoff für diesen Halogen-Fluor-Austausch notwendig wird, kann der Fachmann das einzusetzende Metallhalogenid vorab mit Fluorwasserstoff umsetzen. Die Menge an verbrauchtem Fluorwasserstoff und/oder die Menge an gebildetem Halogenwasserstoff ermöglicht es, zu berechnen, welche Menge an Fluorwasserstoff zusätzlich zu der für die Umsetzung des eingesetzten Halogenkohlenwasserstoffs hinaus benötigt wird.

Ganz besonders einfach und zweckmäßig ist folgende Vorgehensweise: Das Metallhalogenid, beispielsweise Molybdänpentachlorid oder -bromid, Niobpentachlorid oder -bromid oder Tantalpentachlorid oder -bromid wird im Fluorierungsreaktor vorgelegt und mit Fluorwasserstoff versetzt, bis sich kein Chlorwasserstoff bzw. Bromwasserstoff mehr entwickelt. Nach Zusatz des Sulfonsäurederivats leitet man den zu fluorierenden Halogenkohlenwasserstoff und den zu seiner Fluorierung benötigten Fluorwasserstoff in das Katalysatorgemisch ein. Die Zuführung von zusätzlichem Fluorwasserstoff für die Fluorierung des Metallchlorids oder Bromids ist hier nicht notwendig.

Es ist zweckmäßig, zur Herstellung niedrigfluorierter Verbindungen, beispielsweise von $CHCl_2CCl_2F$ aus $CHCl_2CCl_3$ oder $CCl_2=CCl_2$, von $CH_2ClCCl_2F$ aus $CH_2ClCCl_3$ oder $CHCl=CCl_2$ oder von $CH_3CCl_2F$ aus $CH_3CCl_3$ oder $CH_2=CCl_2$, im unteren Temperaturbereich, beispielsweise zwischen 50 und 150 °C zu arbeiten. Der Umsetzungsgrad kann analytisch verfolgt werden, beispielsweise durch Probenentnahme und Untersuchung durch Gaschromatographie.

Vorteilhaft ist hier der hohe Umsetzungsgrad des erfindungsgemäßen Verfahrens.

In der folgenden Tabelle 1 sind, unter Angabe von einsetzbaren Ausgangsverbindungen und der stöchiometrisch notwendigen Menge an Fluorwasserstoff, einige Beispiele für erfindungsgemäß herstellbare Fluor enthaltende Ethanderivate angegeben.

3

## Tabelle 1

$$CCl_2 = CCl_2 + HF \rightarrow CHCl_2CCl_2F$$

$$CCl_2 = CCl_2 + 3HF \rightarrow CHCl_2CF_3$$

$$CHCl_2CCl_3 + 3HF \rightarrow CHCl_2CF_3$$

$$CHCl_2CCl_2F + 2HF \rightarrow CHCl_2CF_3$$

$$CHCl_2CClF_2 + HF \rightarrow CHCl_2CF_3$$

$$CHCl = CCl_2 + 3HF \rightarrow CH_2ClCF_3$$

$$CH_2ClCCl_3 + 3HF \rightarrow CH_2ClCF_3$$

$$CH_2 = CCl_2 + 3HF \rightarrow CH_3CF_3$$

$$CH_3CCl_3 + 3HF \rightarrow CH_3CF_3$$

$$CFH = CF_2 + HF \rightarrow CH_2FCF_3$$

Die Vorteile des erfindungsgemäßen Verfahrens zeigen sich ganz besonders bei der Herstellung höherfluorierter Produkte. Bei der Herstellung höherfluorierter Produkte, beispielsweise der Herstellung von $CHCl_2CF_3$ aus $CHCl_2CCl_3$, $CHCl_2CCl_2F$, $CHCl_2CClF_2$, $CCl_2=CCl_2$ oder deren Gemischen, zur Herstellung von $CH_2ClCF_3$ aus $CH_2ClCCl_3$, $CH_2ClCCl_2F$, $CH_2ClCClF_2$, $CHCl=CCl_2$ oder deren Gemischen oder zur Herstellung von $CH_3CF_3$ aus $CH_3CCl_3$, $CH_3CCl_2F$, $CH_3CClF_2$, $CH_2=CCl_2$ oder deren Gemischen, arbeitet man zweckmäßigerweise im höheren Temperaturbereich, beispielsweise zwischen 70 und 220 °C und 10 bis 50 bar, und verwendet vorzugsweise Fluorsulfonsäure als Sulfonsäurederivat. Auch hier kann der Umsetzungsgrad durch Analyse von gezogenen Proben der Reaktionsmischung ermittelt werden.

Besonders vorteilhaft ist das erfindungsgemäße Verfahren deshalb zur Herstellung höherfluorierter Verbindungen, insbesondere von trifluormethylgruppenhaltigen Ethanderivaten der allgemeinen Formel $F_kH_nCl_{3-k-n}C\text{-}CF_3$ (Ia), in welchen k 0, 1 oder 2 und n 1, 2 oder 3 bedeutet.

Diese bevorzugte Ausführungsform des erfindungsgemäßen Verfahrens ist dadurch gekennzeichnet, daß man zur Herstellung von Fluor enthaltenden Ethanderivaten der allgemeinen Formel (Ia)

$$F_kH_nCl_{3-k-n}C\text{-}CF_3 \qquad (Ia)$$

worin k 0, 1 oder 2 und n 1, 2 oder 3 bedeutet, bei einer Temperatur von 0 bis 250 °C und einem Druck von 1 bis 100 bar (abs.)

a) ein halogeniertes Alken der allgemeinen Formel

$$F_kH_mCl_{2-k-m}C = CX^1X^2 \qquad (II),$$

worin k und m die oben angegebene Bedeutung besitzen, $X^1$ und $X^2$ Fluor, Chlor oder Brom bedeuten, mit Fluorwasserstoff umsetzt, wobei der Fluorwasserstoff in einer Menge in der Reaktionsmischung vorliegt, die der mindestens einfachen Menge der stöchiometrisch zur Fluorwasserstoffanlagerung und zum Halogen-Fluor-Austausch am Alken notwendigen Menge entspricht oder

b) ein halogeniertes Alkan der allgemeinen Formel

$$F_kH_nCl_{3-k-n}C\text{-}CY^1Y^2Y^3 \qquad (III)$$

worin k und n die oben angegebene Bedeutung besitzen und $Y_1$ und $Y_2$ Fluor, Chlor oder Brom und $Y_3$ Chlor oder Brom bedeuten, mit Fluorwasserstoff umsetzt, wobei der Fluorwasserstoff in der Reaktionsmischung in einer Menge vorliegt, die der mindestens einfachen Menge der stöchiometrisch zum Halogen-Fluor-Austausch am Alkan notwendigen Menge entspricht.

Besonders augenfällig werden die Vorteile des erfindungsgemäßen Verfahrens, wenn man von den entsprechenden halogenierten Alkenen als Ausgangsverbindung ausgeht. Der Umsatzgrad bei Anwendung dieser eigentlich sehr reaktionsträgen Verbindungen ist sehr hoch. Die bei anderen Verfahren beobachtete Bildung von Polymerisaten sowie von Verbindungen, die nicht durch Anlagerung von Fluorwasserstoff, sondern durch Anlagerung von Halogen entstanden sind, wird beim erfindungsgemäßen Verfahren allenfalls in verschwindend geringer Menge beobachtet.

Gemäß einer bevorzugten Variante des erfindungsgemäßen Verfahrens setzt man halogenierte Alkene als Ausgangsverbindungen ein. In einem ersten Reaktionsschritt bilden sich zunächst Fluorwasserstoffadditions-Verbindungen. Bezweckt man die Herstellung derartiger niedrig fluorierter Verbindungen, die ja wertvolle Eigenschaften aufweisen, beispielsweise als Lösungsmittel, isoliert man diese Verbindungen aus der Reaktionsmischung. Bezweckt man die Herstellung höherfluorierter Produkte, insbesondere von trifluormethylgruppenhaltigen Ethanderivaten, kann man diese Verbindungen isolieren und weiter fluorieren. Zweckmäßigerweise isoliert man diese intermediär in einen ersten Reaktionsschritt entstehenden Fluorwasserstoffadditions-Verbindungen nicht, sondern setzt diese Verbindungen in situ mit weiterem Fluorwasserstoff zu den gewünschten höher fluorierten Produkten um.

Man kann auch von mindestens teilweise fluorierten Alkenen ausgehen. So kann gemäß einer bevorzugten Ausführungsform des erfindungsgemäßen Verfahrens $CH_2FCF_3$ hergestellt werden, indem Trifluorethylen mit Fluorwasserstoff in Anwesenheit des Katalysatorsystems aus Metallhalogenid und Sulfonsäurederivat umgesetzt wird. Erstaunlicherweise wird eine Polymerisation des Alkens nicht beobachtet.

Hervorragend geeignet ist das erfindungsgemäße Verfahren zur Herstellung von $CHCl_2CF_3$ (R123). Diese ganz besonders bevorzugte Ausführungsform des erfindungsgemäßen Verfahrens ist dadurch gekennzeichnet, daß man zur Herstellung von $CHCl_2CF_3$

a) $CCl_2 = CCl_2$ mit Fluorwasserstoff umsetzt, wobei die Menge an Fluorwasserstoff in der Reaktionsmischung der mindestens einfachen Menge entspricht, die stöchiometrisch zur Fluorwasserstoff-Anlagerung und zum Chlor-Fluor-Austausch am Alken notwendigen Menge entspricht, oder

b) $CHCl_2CCl_3$, $CHCl_2CFCl_2$, $CHCl_2CF_2Cl$

oder deren Gemische mit Fluorwasserstoff umsetzt, wobei die Menge an Fluorwasserstoff in der Reaktionsmischung der mindestens einfachen Menge entspricht, die stöchiometrisch zum Chlor-Fluor-Austausch am Alkan notwendig ist.

Ganz besonders bevorzugt ist die Herstellung von $CHCl_2CF_3$ aus $CCl_2 = CCl_2$ nach Variante a). Das Mol-Verhältnis von $CCl_2 = CCl_2$ und Fluorwasserstoff liegt vorteilhaft zwischen 1:3 und 1:100.

Weiter oben wurde bereits festgestellt, daß im erfindungsgemäßen Verfahren das Mol-Verhältnis von Metallhalogenid zu Sulfonsäurederivat zwischen 100:1 und 1:10 liegen muß. Steigt der Anteil an Sulfonsäurederivat im Katalysatorgemisch über diesen Wert, sinkt besonders im Hinblick auf die Herstellung von trifluormethylgruppenhaltigen Ethanderivaten die Selektivität. Sinkt der Anteil an Sulfonsäurederivat im Katalysatorgemisch unter diesen Wert, sinkt der Umsatz zu höher fluorierten Produkten auf unakzeptable Werte ab.

Vorteilhafterweise liegt das Mol-Verhältnis von Metallhalogenid zu Sulfonsäurederivat zwischen 10:1 und 1:3, besonders vorteilhaft zwischen 2:1 und 1:2.

Das Mol-Verhältnis von Ausgangsverbindung zum Katalysatorgemisch liegt, wie weiter oben angeführt, zwischen 10:1 und 1:100. Hierbei wird die Mol-Zahl des Katalysatorgemisches durch Addition der Mol-Zahlen von Metallhalogenid und Sulfonsäurederivat berechnet. Setzt man beispielsweise 1 Mol Ausgangsverbindung in Anwesenheit eines Gemisches von 0,5 Mol Niobpentahalogenid und 0,5 Mol Fluorsulfonsäure mit Fluorwasserstoff um, so beträgt das Mol-Verhältnis von Ausgangsverbindung zu Katalysatorgemisch in diesem Falle 1:1.

Bevorzugt liegt das Mol-Verhältnis von Ausgangsverbindung zu Katalysatorgemisch zwischen 10:1 und 1:10, besonders bevorzugt zwischen 2:1 und 1:5.

Als Sulfonsäurederivat setzt man vorzugsweise Fluorsulfonsäure oder Trifluormethansulfonsäure, insbesondere bevorzugt Fluorsulfonsäure ein.

Als Metallhalogenid setzt man bevorzugt Niobpentahalogenid, Tantalpentahalogenid oder deren Gemische ein, wobei Halogenid Fluorid, Chlorid oder Bromid bedeutet. Besonders bevorzugt setzt man als Metallhalogenid Niobpentachlorid oder Tantalpentachlorid ein.

Ausgezeichnete Ergebnisse wurden erhalten bei Anwendung von Katalysatorgemischen, umfassend Niobpentachlorid oder Tantalpentachlorid und Fluorsulfonsäure.

Eine ganz besonders bevorzugte Ausführungsform des Verfahren ist dadurch gekennzeichnet, daß man zur Herstellung von $CHCl_2CF_3$ ausgeht von $CCl_2 = CCl_2$, dieses mit der mindestens einfachen der stöchiometrisch zur Fluorwasserstoffanlagerung und zum Chlor-Fluor-Austausch am Alken notwendigen Menge an Fluorwasserstoff umsetzt, wobei man als Katalysatorgemisch Gemische von Niobpentahalogenid, Tantalpentahalogenid oder Gemische dieser Verbindungen mit Fluorsulfonsäure einsetzt.

Feuchtigkeit wirkt sich im erfindungsgemäßen Verfahren störend aus. Die Umsetzung wird daher zweckmäßigerweise unter Bedingungen durchgeführt, die verhindern, daß eine schädliche Menge an Wasser in die Reaktionsmischung gelangen kann. Man verwendet im wesentlichen wasserfreien Fluorwasserstoff. Je nach eingesetzter Menge an Fluorwasserstoff kann es empfehlenswert sein, den handelsübli-

EP 0 451 746 B1

chen Fluorwasserstoff vor seiner Verwendung zu trocknen. Weiterhin ist es empfehlenswert, die verwendete Apparatur im möglichst trockenen Zustand zu halten. Hierzu kann man Leitungen, Reaktionsgefäße, Einrichtungen zur Produktaufarbeitung und -Aufbewahrung mit trockenen Gasen spülen, beispielsweise mit trockener Luft oder trockenem Stickstoff-Gas.

Die Umsetzung kann im batch-Verfahren oder kontinuierlich durchgeführt werden.

Die Aufarbeitung kann durch Leiten der Reaktionsprodukte durch einen Gaswäscher und anschließende fraktionierte Destillation erfolgen.

Die für die Durchführung verwendete Apparatur sollte gegenüber Fluorwasserstoff, Metallhalogeniden und dem jeweils verwendeten Sulfonsäurederivat resistent sein. Zweckmäßig verwendet man Bauteile aus Teflon und speziellen Legierungen wie "Hastelloy", einer fluorwasserstoffresistenten Nickellegierung.

Ein weiterer Gegenstand der vorliegenden Erfindung ist das im erfindungsgemäßen Verfahren verwendbare Katalysatorgemisch. Das erfindungsgemäße Katalysatorgemisch umfaßt ein Gemisch von Metallhalogenid und einem Sulfonsäurederivat im Mol-Verhältnis von 100:1 bis 1:10, wobei das Metallhalogenid ausgewählt ist aus der Niobpentahalogenid, Tantalpentahalogenid und Molybdänpentahalogenid oder deren Gemische umfassenden Gruppe und das Sulfonsäurederivat ausgewählt wird aus der Fluorsulfonsäure, Perfluorniedrigalkansulfonsäuren mit 1 bis 4 C-Atomen, insbesondere Trifluormethansulfonsäure, umfassenden Gruppe.

Dieses Katalysatorgemisch kann der Fachmann durch einfaches Vermischen der Bestandteile herstellen.

Ein bevorzugtes erfindungsgemäßes Katalysatorgemisch ist erhältlich durch Vermischen von Molybdän-, Tantal- oder Niobpentahalogenid, insbesondere der Chloride oder Bromide mit Sulfonsäurederivat und Fluorwasserstoff. Der Fluorwasserstoff wird zweckmäßigerweise in einer solchen Menge zugemischt, daß kein Chlorwasserstoff oder Bromwasserstoff mehr freigesetzt wird.

Ein besonders bevorzugtes Katalysatorgemisch umfaßt Metallhalogenid und Sulfonsäurederivat im Mol-Verhältnis von 10:1 bis 1:3, insbesondere 2:1 bis 1:2.

Ganz besonders bevorzugte Katalysatorgemische umfassen ein Gemisch von Niobpentahalogenid, Tantalpentahalogenid oder deren Gemischen als Metallhalogenid und Fluorsulfonsäure, Trifluormethansulfonsäure oder deren Gemischen als Sulfonsäurederivat.

Hervorragende Ergebnisse bei der Anwendung des erfindungsgemäßen Verfahren ergeben Katalysatorgemische, welche ein Gemisch von Niobpentahalogenid oder Tantalpentahalogenid und Fluorsulfonsäure umfassen.

Ganz besonders bevorzugte erfindungsgemäße Katalysatorgemische sind erhältlich durch Vermischen von Niobpentachlorid oder Tantalpentachlorid und Fluorsulfonsäure und Versetzen dieses Gemisches bis zur Beendigung der Chlorwasserstofffreisetzung.

Ein weiterer Gegenstand der Erfindung sind Fluorwasserstofflösungen zur Anwendung im erfindungsgemäßen Verfahren, welche 0,01 bis 99,99 Gew.-%, bevorzugt 10 bis 90 Gew.-%, insbesondere 30 bis 70 Gew.-% des erfindungsgemäßen Katalysatorgemisches enthalten. Diese Fluorwasserstofflösungen sind erhältlich durch Vermischen des Metallhalogenids und des Sulfonsäurederivats in der notwendigen Menge Fluorwasserstoff und gewünschtenfalls Abtrennen etwaig gebildeten Chlorwasserstoffs oder Bromwasserstoffs.

Die durch das erfindungsgemäße Verfahren erhaltenen fluorhaltigen Ethanderivate sind wertvolle umweltverträgliche Lösungsmittel, Treibmittel und Zwischenprodukte für die chemische Synthese.

Das erfindungsgemäße Verfahren zeichnet sich aus durch hohen Umsatz, hohe Selektivität, und es kann vorteilhafterweise in der flüssigen Phase durchgeführt werden.

Die hohe Wirksamkeit des erfindungsgemäßen Verfahrens muß als überraschend und unvorhersehbar angesehen werden. Setzt man nämlich beispielsweise Perchlorethylen als Ausgangsverbindung und reine Trifluormethansulfonsäure als Katalysator ein, ist der Umsatz gering, und man beobachtet teilweise Polymerisation der Ausgangsverbindung. Bei Anwendung von Fluorsulfonsäure als Katalysator ist der Umsatz verschwindend gering. Molybdänpentachlorid als Katalysator ergibt zwar einen recht hohen Umsatzgrad. Die Bildung von höher fluorierten Produkten wie R123 erfolgt jedoch nur in sehr geringem Ausmaß, außerdem ist der teure Katalysator bereits nach einmaliger Versuchsdurchführung unbrauchbar. Niobpentachlorid als Katalysator liefert geringere Umsatzgrade. Es ist daher völlig unvorhersehbar, daß die Kombination dieser für sich allein fast nicht brauchbaren Kataslysatorbestandteile im erfindungsgemäßen Verfahren zu derartig guten Ergebnissen bezüglich Umsatzgrad, Selektivität und Lebensdauer des Katalysatorgemisches führt und auch die Herstellung höher fluorierter Produkte wie insbesondere R123 gestattet.

Die folgenden Beispiele sollen das erfindungsgemäße Verfahren weiter erläutern, ohne es in seinem Umfang einzuschränken.

Beispiele

Die Versuche der Beispiele 1 bis 6 wurden in der gleichen Vorrichtung durchgeführt. Die Reaktion wurde durchgeführt in einem Laborautoklaven aus V4A-Stahl (ein mit Chrom, Nickel und Molybdän legierter Stahl). Das Innenvolumen dieses Autoklaven betrug 0,25 Liter. Der Autoklav war mit einem Magnetrührer, einem Tauchrohr, über welches die Ausgangsverbindungen eindosiert werden konnten, und einem Thermostutzen, über welchen die Innentemperatur gemessen werden konnte, ausgerüstet. Der Laborautoklav wies weiterhin einen Gasauslaß auf, welcher mit einem mit Wasser gefüllten Gaswäscher verbunden war. Der Gaswäscher wiederum war mit einer Tiefkältekondensationseinrichtung verbunden.

Allgemeine Vorschrift zur Versuchsdurchführung für die Beispiele 1 bis 5:

Das Metallhalogenid, das Sulfonsäurederivat und der eingesetzte Halogenkohlenwasserstoff wurden mit Hilfe einer Dosierpumpe über das Tauchrohr in den Autoklaven eingebracht. Anschließend wurde über das Tauchrohr auch der Fluorwasserstoff zudosiert. Es wurde sofort eine Chlorwasserstoffentwicklung beobachtet, vermutlich resultierend aus der Umsetzung des eingesetzten Metallchlorids und Fluorwasserstoff. Es bildete sich eine Fluorwasserstofflösung, welche das Katalysatorgemisch enthielt. Der freigesetzte Chlorwasserstoff wurde aus dem Laborautoklaven abgeleitet und in den Gaswäscher geleitet, wo er vom dort vorhandenen Wasser absorbiert wurde.

Sobald die anfänglich zu beobachtende Chlorwasserstoffentwicklung nachließ, wurde der Laborautoklav verschlossen, bis zur in den Beispielen angegebenen maximalen Temperatur aufgeheizt, dann auf Umgebungstemperatur gebracht, entstandenes Chlorwasserstoff-Gas abgelassen, der Autoklav verschlossen und dann wiederum bis zur maximalen Temperatur aufgeheizt. Dieses Aufheizen wurde mittels eines Ölbades bewirkt. Die maximale Temperatur wurde dann 5 Stunden beibehalten. Anschließend wurde der Laborautoklav auf Raumtemperatur (etwa 22 °C) abgekühlt. Der Autoklaveninhalt wurde dann auf Umgebungsdruck gebracht und flüchtige Bestandteile durch den Gaswäscher geleitet. Im Gaswäscher wurde vorhandener Chlorwasserstoff und Fluorwasserstoff ausgewaschen. Das den Gaswäscher verlassende, im wesentlichen aus organischen Verbindungen bestehende Rohgas wurde gaschromatographisch untersucht. Das den Gaswäscher verlassende Rohgas wurde in eine Tiefkältekondensationsanlage überführt. Das Rohprodukt konnte gewünschtenfalls durch destillative Aufarbeitung weiter aufgetrennt werden.

Beispiel 1:

Herstellung von Trifluordichlorethan und Difluortrichlorethan

In den Autoklaven wurden mit Hilfe der Dosierpumpe 27 g Niobpentachlorid (0,1 mol), 15 g Trifluormethansulfonsäure (0,1 mol), 40 g Tetrachlorethylen (0,24 mol) und 40 g Fluorwasserstoff (2,0 mol) eingebracht. Nach Beendigung der anfänglich zu beobachtenden Chlorwasserstoffentwicklung wurde der Autoklav verschlossen und auf eine maximale Temperatur von 160 °C erhitzt. Der Druck stieg dabei bis auf 33 bar absolut (abs.) an. Nach dem Abkühlen wurde der Autoklaveninhalt wie oben beschrieben aufgearbeitet. Die Untersuchung des verbleibenden, nichtflüchtigen Reaktionsrückstandes ergab, daß das Tetrachlorethylen zu 100 % umgesetzt worden war.

Die gaschromatische Untersuchung der Gasphase ergab folgende Werte:

3,1 Gew.-% $C_2HF_5$; 0,6 Gew.-% $C_2HClF_4$; 50,2 Gew.-% $C_2HCl_2F_3$; 44,3 Gew.-% $C_2HCl_3F_2$; 0,8 Gew.-% $C_2Cl_4F_2$.

Das Beispiel zeigt, daß mittels Niobpentachlorid und Trifluormethansulfonsäure als Katalysatorgemisch Tetrachlorethylen in höher fluorierte Ethane, insbesondere Trifluordichlorethan und Difluortrichlorethan unter den angegebenen Bedingungen überführt werden kann.

Beispiel 2:

Herstellung von Trifluordichlorethan

In den Laborautoklaven wurden 27 g Niobpentachlorid (0,1 mol), 10 g Fluorsulfonsäure (0,1 mol), 40 g Tetrachlorethylen (0,24 mol) und 40 g Fluorwasserstoff (2,0 mol) eingebracht. Nach Ablassen des anfänglich entstehenden Chlorwasserstoffs wurde der Autoklav verschlossen, bis zu einer maximalen Temperatur von 130 °C erhitzt, wobei der Druck bis auf 19 bar absolut anstieg. Nach dem Abkühlen wurde der Autoklav auf Normaldruck gebracht und flüchtige Bestandteile abgetrennt. Die Untersuchung des nicht flüchtigen Rückstandes ergab, daß das eingesetzte Tetrachlorethylen zu 99,9 Gew.-% umgesetzt war. Die flüchtigen Bestandteile wurden durch einen Gaswäscher geleitet und das den Gaswäscher verlassende Rohprodukt gaschromatographisch untersucht. Die Untersuchung ergab folgende Anteile:

0,4 Gew.-% $C_2HF_5$; 0,1 Gew.-% $CHF_3$; 0,8 Gew.-% $C_2HClF_4$; 81,2 Gew.-% $C_2HCl_2F_3$; 16,7 Gew.-% $C_2HCl_3F_2$.

Dieses Beispiel zeigt, daß bei Anwendung von Niobpentachlorid und Fluorsulfonsäure als Katalysatorgemisch Tetrachlorethylen in guter Ausbeute und mit guter Selektivität in Trifluordichlorethan überführt wird.

Beispiel 3:

Herstellung von Trifluordichlorethan aus Difluortrichlorethan

In den Laborautoklaven wurden 5 g Niobpentachlorid (0,02 mol), 14 g Fluorsulfonsäure (0,14 mol), 33 g Difluortrichlorethan (0,16 mol) und 40 g Fluorwasserstoff (2,0 mol) eingebracht. Der zunächst entstehende Chlorwasserstoff wurde wiederum abgelassen. Nach Abklingen dieser anfänglichen Chlorwasserstoffbildung wurde der Laborautoklav verschlossen und auf eine maximale Temperatur von 125 °C erhitzt. Der Druck stieg dabei bis auf 25 bar absolut an. Nach dem Abkühlen wurde der Autoklav auf Normaldruck gebracht und flüchtige Bestandteile abgetrennt. Die flüchtigen Bestandteile wurden durch den Gaswäscher geleitet und das den Gaswäscher verlassende Rohprodukt wurde gaschromatographisch analysiert. Es ergaben sich folgende Werte:

0,3 Gew.-% $C_2H_2Cl_2F_2$; 66,7 Gew.-% $C_2HCl_2F_3$; 2,5 Gew.-% $C_2Cl_3F_3$; 30,5 Gew.-% $C_2HCl_3F_2$

Der Umsatz an Difluortrichlorethan wurde zu 49,1 Gew.-% bestimmt.

Beispiel 4:

Herstellung von Trifluordichlorethan und Difluortrichlorethan aus Fluortetrachlorethan

In den Autoklaven wurden 5 g Niobpentachlorid (0,02 mol), 14 g Fluorsulfonsäure (0,14 mol), 36 g Fluortetrachlorethan (0,16 mol) und 40 g Fluorwasserstoff (2,0 mol) eingebracht. Der zunächst entstehende Chlorwasserstoff wurde abgelassen, der Autoklav verschlossen und auf eine maximale Temperatur von 130 °C erhitzt. Der Druck stieg dabei bis auf 22 bar absolut an. Der Autoklav wurde auf Raumtemperatur abgekühlt und auf Umgebungsdruck gebracht. Die flüchtigen Bestandteile wurden durch den Gaswäscher geleitet und das Rohprodukt gaschromatisch analysiert. Es ergaben sich folgende Werte:

1,7 Gew.-% $CHF_3$; 2,9 Gew.-% $CHClF_2$; 0,6 Gew.-% $C_2H_2Cl_2F_2$; 46,4 Gew.-% $C_2HCl_2F_3$; 0,8 Gew.-% $C_2Cl_3F_3$; 47,6 Gew.-% $C_2HCl_3F_2$.

Der Umsatzgrad wurde zu 63,6 Gew.-% bestimmt.

Die Beispiele 3 und 4 demonstrieren die Eignung des erfindungsgemäßen Verfahrens zur Herstellung höher fluorierter Halogenalkane aus entsprechend niedriger fluorierten Halogenalkanen.

Beispiel 5:

Herstellung von Trifluorchlorethan aus Trichlorethylen

In den Autoklaven wurden 36 g Tantalpentachlorid (0,1 mol) 10 g Fluorsulfonsäure (0,1 mol), 32 g Trichlorethylen (0,24 mol) und 40 g Fluorwasserstoff (2,0 mol) eingebracht. Wiederum wurde der zunächst entstehende Chlorwasserstoff abgelassen. Nach Abklingen der Chlorwasserstoffbildung wurde der Autoklav verschlossen, auf eine maximale Temperatur von 140 °C gebracht, wobei der Druck bis auf 43 bar absolut anstieg. Nach dem Abkühlen wurde der Autoklav auf Umgebungsdruck gebracht und flüchtige Bestandteile durch den Gaswäscher geleitet. Das den Gaswäscher verlassende Rohprodukt wurde gaschromatographisch analysiert. Es ergaben sich folgende Werte:

89,0 Gew.-% $C_2H_2ClF_3$; 7,9 Gew.-% $CCl_3F$, 0,4 Gew.-% $C_2HCl_2F_3$; 0,4 Gew.-% $C_2H_2Cl_2F_2$; 2,3 Gew.-% Unbekannte.

Der Trichlorethylenumsatz betrug 100 Gew.-%.

Beispiel 6:

Halbkontinuierliche Herstellung von Trifluordichlorethan

Bei diesem Beispiel wurde die oben beschriebene Apparatur verwendet. In den Autoklaven wurden vor der ersten Aufheizphase 36 g Tantalpentachlorid (0,1 mol), 10 g Fluorsulfonsäure (0,1 mol), 40 g Fluorwasserstoff (2 mol) und 40 g Tetrachlorethylen (0,24 mol) eingebracht. Der zunächst entstehende Chlorwasserstoff wurde abgelassen. Es lag dann wieder eine Fluorwasserstofflösung vor, die das Katalysatorgemisch enthielt. Der Autoklav wurde dann verschlossen und der ersten Aufheizphase unterworfen. Hierzu wurde der Autoklaveninhalt auf die in Tabelle 2 angegebene Temperatur gebracht und während der in Tabelle 2 angegebenen Zeitdauer bei dieser Temperatur gehalten. Anschließend wurde der Autoklaveninhalt auf Umgebungstemperatur gebracht und entstandener Chlorwasserstoff abgelassen. Anschließend wurde eine Probe der bei Umgebungstemperatur und Normaldruck flüchtigen Verbindungen entnommen und im Hinblick auf die darin enthaltenen organischen Verbindungen gaschromatographisch untersucht. Die Analysenwerte, angegeben in Gew.-%, finden sich in Tabelle 2.

Anschließend wurde der Autoklaveninhalt der zweiten Aufheizphase unterworfen. Nach Abkühlen wurde der Reaktor auf Normaldruck gebracht und diesesmal der gesamte Gehalt der bei Umgebungstemperatur und Normaldruck flüchtigen Verbindungen aus dem Reaktor abgelassen und durch den Gaswäscher geleitet. Das den Gaswäscher verlassende Rohprodukt wurde gaschromatographisch untersucht.

In den Reaktor, in welchem neben schwerflüchtigen organischen Verbindungen auch das Katalysatorgemisch verblieben war, wurde dann erneut 40 g Tetrachlorethylen und 40 g Fluorwasserstoff eingebracht. Der Reaktor wurde dann der dritten Aufheizphase unterworfen, abgekühlt, entstandener Chlorwasserstoff wurde abgelassen und anschließend eine Probe der bei Umgebungstemperatur und Normaldruck flüchtigen organischen Verbindungen analysiert. Daraufhin wurde der Reaktor der vierten Aufheizphase unterworfen, abgekühlt, und der gesamte Anteil an bei Umgebungstemperatur und Normaldruck flüchtigen Verbindungen aus dem Reaktor in den Gaswäscher geleitet, worauf das den Gaswäscher verlassene Rohprodukt wiederum gaschromatographisch untersucht wurde.

Auf diese Weise wurden insgesamt 10 Aufheizphasen durchgeführt. Nach der ersten, dritten, fünften, siebten und achten Aufheizphase wurde aus dem Reaktor jeweils nur der entstandene Chlorwasserstoff abgelassen und Proben zur Analyse entnommen. Nach der zweiten, vierten, sechsten und neunten Aufheizphase wurde jeweils der gesamte Anteil der bei Umgebungstemperatur und Normaldruck flüchtigen Verbindungen des Reaktorinhalts aus dem Reaktor abgelassen, durch den Gaswäscher geleitet und das den Gaswäscher verlassende Rohprodukt analysiert.

Entsprechend wurden nach der zweiten, vierten, sechsten und neunten Aufheizphase jeweils 40 g Fluorwasserstoff und jeweils 40 g Tetrachlorethylen in den Reaktor eingebracht. Das Katalysatorgemisch wurde während des gesamten Versuchs weder ergänzt oder aufgefrischt noch regeneriert.

Die in jeder Aufheizphase eingestellte maximale Temperatur, der erreichte maximale Druck und die Zeitdauer, während der die maximale Temperatur aufrechterhalten wurde, sowie die Daten der gaschromatographischen Analyse der nach jeder Aufheizphase erhaltenen Gasphase sind in Tabelle 2 zusammengestellt.

## Tabelle 2

| Auf-heiz-phase | Temp. max. | Druck max. | Dauer | $C_2H_2Cl_2F_2$ | $C_2H_2ClF_3$ | $C_2HCl_3F_2$ | $C_2HCl_2F_3$ |
|---|---|---|---|---|---|---|---|
| 1 | 120 °C | 39 bar | 3.0 h | 0,1 % | 0,2 % | 3,6 % | 94,7 % |
| 2 | 130 °C | 14 bar | 5.0 h | - | 0,2 % | 2,3 % | 97,0 % |
| 3 | 120 °C | 35 bar | 5.0 h | - | - | 6,1 % | 92,7 % |
| 4 | 120 °C | 17 bar | 6.0 h | 0,1 % | 0,1 % | 4,0 % | 95,2 % |
| 5 | 120 °C | 34 bar | 7.5 h | 0,2 % | 0,1 % | 14,4 % | 84,2 % |
| 6 | 120 °C | 27 bar | 7.0 h | 0,1 % | 0,1 % | 21,1 % | 82,4 % |
| 7 | 90 °C | 10 bar | 4.0 h | - | - | 10,0 % | 88,9 % |
| 8 | 105 °C | 11 bar | 6.0 h | - | - | 8,3 % | 90,9 % |
| 9 | 110 °C | 11 bar | 6.0 h | - | - | 18,1 % | 81,2 % |
| 10 | 110 °C | 11 bar | 7.0 h | 3,5 % | - | 16,5 % | 79,8 % |

"%" bedeutet "Gew.-%"

Das gebildete Dichlortrifluorethan bestand aus R123 sowie Spuren von R123a.

Nach Beendigung der 10. Aufheizphase betrug der Gesamtumsatz an Tetrachlorethylen 91 Gew.-%. Im organischen Teil des Reaktionsrückstandes (48 g) fanden sich 0,8 Gew.-% Trifluordichlorethan, 37,6 Gew.-% Difluortrichlorethan, 37,6 Gew.-% Tetrachlorethylen sowie 23,9 Gew.-% Fluortetrachlorethan.

Dieses Beispiel demonstriert die Eignung des erfindungsgemäßen Verfahrens zur halbkontinuierlichen und kontinuierlichen Verfahrensweise. Der Anteil höherfluorierter Ethane, insbesondere Trifluordichlorethan sowie Difluortrichlorethan ist aufgrund der Standfestigkeit des Katalysatorgemisches auch nach langer Reaktionsdauer noch sehr gut.

Beispiel 7:

Herstellung von 1,1,1,2-Tetrafluorethan (R134a) aus Trifluorethylen und Fluorwasserstoff.

Die in diesem Beispiel verwendete Apparatur entsprach im Prinzip der in den Beispielen 1 bis 6 verwendeten Apparatur. Da das verwendete Ausgangsprodukt Trifluorethylen bei Normalbedingungen gasförmig ist, wurde es nicht über eine Dosierpumpe in den Autoklaven eingebracht. Das in den Autoklaven führende Tauchrohr war über absperrbare Ventile mit einer Trifluorethylen enthaltenden Druckgasflasche verbunden. Aus dieser Druckgasflasche konnte das Trifluorethylen bei entsprechendem Öffnen der Ventile in den Autoklaven gasförmig eingebracht werden.

In den Autoklaven wurden zunächst 39,8 g Tantalpentachlorid (0,11 mol) sowie Fluorwasserstoff eingebracht und unter quantitativer Bildung von Tantalpentafluorid umgesetzt. Dann wurden 23,6 g Fluorsulfonsäure ($HSO_3F$) (0,23 mol) zugegeben.

Dann wurden in den Autoklaven 72 g Fluorwasserstoff (3,6 mol) flüssig zugegeben und 18 g Trifluorethylen (0,22 mol) aufgepresst. Der Autoklav wurde dann verschlossen und über einen Zeitraum von 2 Stunden bei 110 °C gehalten. Der Überdruck betrug 15 bis 17 Atmosphären.

Gasförmige Reaktionsprodukte wurden dann durch den Wäscher geführt und unmittelbar durch Gaschromatographie, kombiniert mit Massenspektrometrie (GC-MS-Analyse) analysiert.

Dabei wurde gefunden, daß die gasförmigen Reaktionsprodukte zu 99,5 % aus dem gewünschten Produkt Tetrafluorethan (R134a) bestanden. Weiterhin waren 0,1 % Wasser (aus dem Wäscher mitgerissen)

und 0,4 % $C_2F_3H_3$ (gleiche Mengen von R143 und R143a) enthalten.

Der Reaktorinhalt wurde dann hydrolytisch aufgearbeitet. Polymere Produkte des Trifluorethylens wurden nicht gefunden.

Dieses Beispiel zeigt, daß auch Trifluorethylen nach dem erfindungsgemäßen Verfahren mit Fluorwasserstoff zu R134a umgesetzt werden kann, ohne daß die Bildung von Polymeren zu befürchten ist. Die Reinheit des Rohprodukts ist bereits derartig hoch, daß für die meisten Anwendungszwecke eine weitere Reinigung überflüssig wäre.

Beispiel 8:

Herstellung von 1,1,1,2-Tetrafluorethan (R134a) aus Trifluorethylen und Fluorwasserstoff.

Die Vorgehensweise entsprach der in Beispiel 7 beschriebenen Vorgehensweise. Der Überdruck betrug diesmal nur etwa 14 Atmosphären, die Reaktionstemperatur 103 °C und die Zeitdauer der Umsetzung etwa 1 Stunde.

| Ansatz: | 20,8 g (0,25 mol) Trifluorethylen |
| | 76 g (3,8 mol) Fluorwasserstoff |
| | Katalysator: wie in Beispiel 7 |
| GC-MS-Analyse: | 2,1 % Wasser (aus dem Wäscher mitgerissen) |
| | 1,6 % 143a |
| | 0,7 % 143 |
| | 95,6 % R134a |

Auch bei kürzerer Reaktionszeit und milderen Bedingungen ist die Reinheit des Produkts hervorragend.

**Patentansprüche**

1. Verfahren zur Herstellung von Fluor enthaltenden Ethanderivaten der allgemeinen Formel (I)

$$F_kH_nCl_{3-k-n}C-CZ^1Z^2F \qquad (I)$$

worin $Z^1$ und $Z^2$ gleich oder verschieden sein können und Wasserstoff, Fluor, Chlor oder Brom bedeuten, k 0, 1 oder 2 und n 1, 2 oder 3 bedeuten

durch Umsetzung eines halogenierten Alkens oder halogenierten Alkans als Ausgangsverbindung mit Fluorwasserstoff in Anwesenheit eines Katalysatorsystems in flüssiger Phase bei einer Temperatur zwischen 0 und 250 °C, wobei der Fluorwasserstoff mindestens in äquimolarer Menge, bezogen auf die Ausgangsverbindung, in der flüssigen Phase vorhanden ist, das Mol-Verhältnis von Ausgangsverbindung zu Katalysatorsystem zwischen 10:1 bis 1:100 beträgt, das Katalysatorsystem ein Gemisch aus Metallhalogenid und einem Sulfonsäurederivat im Mol-Verhältnis von Metallhalogenid zu Sulfonsäurederivat zwischen 100:1 und 1:10 umfaßt, wobei das Metallhalogenid ausgewählt ist aus Niobpentahalogenid, Tantalpentahalogenid, Molybdänpentahalogenid oder deren Gemischen und das Sulfonsäurederivat ausgewählt ist aus der Fluorsulfonsäure, Perfluorniedrigalkansulfonsäuren mit 1 bis 4 C-Atomen, insbesondere Trifluormethansulfonsäure umfassenden Gruppe und wobei man

a) als halogeniertes Alken eine Verbindung der allgemeinen Formel (II) verwendet

$$F_kH_mCl_{2-k-m}C = CX^1X^2 \qquad (II)$$

worin $X^1$ und $X^2$ gleich oder verschieden sein können und Wasserstoff, Fluor, Chlor oder Brom bedeuten, k die oben angegebene Bedeutung besitzt und m 0, 1 oder 2 bedeutet, oder

b) man als halogeniertes Alkan eine Verbindung der allgemeinen Formel (III) verwendet

$$F_kH_nCl_{3-k-n}C-CY^1Y^2Y^3 \qquad (III)$$

worin k und n die obige Bedeutung besitzen, $Y^1$, $Y^2$ gleich oder verschieden sein können und Wasserstoff, Fluor, Chlor oder Brom und $Y^3$ Chlor oder Brom bedeuten.

2. Verfahren nach Anspruch 1, dadurch gekennzeichnet, daß man bei einer Temperatur zwischen 50 und 250 °C und einem Druck von 1 bis 100 bar (abs.) arbeitet.

3. Verfahren nach Anspruch 2, dadurch gekennzeichnet, daß man zur Herstellung von Fluor enthaltenden Ethanderivaten der allgemeinen Formel (Ia)

$F_kH_nCl_{3-k-n}C\text{-}CF_3$     (Ia)

worin k 0, 1 oder 2 und n 1, 2 oder 3 bedeutet, bei einer Temperatur von 50 bis 250 °C und einem Druck von 1 bis 100 bar

a) ein halogeniertes Alken der allgemeinen Formel

$F_kH_mCl_{2-k-m}C = CX^1X^2$     (II)

worin k und m die oben angegebene Bedeutung besitzen, $X^1$ und $X^2$ Fluor, Chlor oder Brom bedeuten, mit Fluorwasserstoff umsetzt, wobei der Fluorwasserstoff in einer Menge in der Reaktionsmischung vorliegt, die der mindestens einfachen Menge der stöchiometrisch zur Fluorwasserstoffanlagerung und zum Halogen-Fluor-Austausch beim Alken notwendigen Menge entspricht oder

b) ein halogeniertes Alkan der allgemeinen Formel

$F_kH_nCl_{3-k-n}C\text{-}CY^1Y^2Y^3$     (III)

worin k und n die oben angegebene Bedeutung besitzen, $Y^1$, $Y^2$ Fluor, Chlor oder Brom und $Y^3$ Chlor oder Brom bedeuten, mit Fluorwasserstoff umsetzt, wobei der Fluorwasserstoff in der Reaktionsmischung in einer Menge vorliegt, die der mindestens einfachen Menge der stöchiometrisch zum Halogen-Fluor-Austausch beim Alkan notwendigen Menge entspricht.

4. Verfahren nach Anspruch 3, dadurch gekennzeichnet, daß man zur Herstellung von $CHCl_2CF_3$
a) $CCl_2 = CCl_2$ mit Fluorwasserstoff umsetzt, wobei die Menge an Fluorwasserstoff in der Reaktionsmischung der mindestens einfachen Menge entspricht, die stöchiometrisch zur Fluorwasserstoff-Anlagerung und zum Chlor-Fluor-Austausch am Alken notwendig ist, oder
b) $CHCl_2CCl_3$, $CHCl_2CFCl_2$, $CHCl_2CF_2Cl$ oder deren Gemische mit Fluorwasserstoff umsetzt, wobei die Menge an Fluorwasserstoff in der Reaktionsmischung der mindestens einfachen Menge entspricht, die stöchiometrisch zum Chlor-Fluor-Austausch am Alkan notwendig ist.

5. Verfahren nach einem der vorhergehenden Ansprüche, dadurch gekennzeichnet, daß man von einem halogenierten Alken ausgeht.

6. Verfahren nach Anspruch 4 oder 5, dadurch gekennzeichnet, daß man zur Herstellung von $CHCl_2CF_3$ ausgeht von $CCl_2 = CCl_2$.

7. Verfahren nach Anspruch 6, dadurch gekennzeichnet, daß das Mol-Verhältnis von $CCl_2 = CCl_2$ und Fluorwasserstoff zwischen 1:3 und 1:100 liegt.

8. Verfahren nach Anspruch 3, dadurch gekennzeichnet, daß man bei einer Temperatur von 80 bis 210 °C und einem Druck von 10 bis 40 bar (abs.) arbeitet.

9. Verfahren nach einem der vorhergehenden Ansprüche, adurch gekennzeichnet, daß man das Metallhalogenid und das Sulfonsäurederivat im Mol-Verhältnis zwischen 10:1 bis 1:3 einsetzt.

10. Verfahren nach einem der vorhergehenden Ansprüche, dadurch gekennzeichnet, daß das Mol-Verhältnis von Ausgangsverbindung zum Katalysatorgemisch zwischen 10:1 und 1:10 liegt.

11. Verfahren nach einem der vorhergehenden Ansprüche, dadurch gekennzeichnet, daß man als Sulfonsäurederivat Fluorsulfonsäure verwendet.

12. Verfahren nach Anspruch 11, dadurch gekennzeichnet, daß man als Katalysatorgemisch Gemische von Niobpentahalogenid, Tantalpentahalogenid oder Gemische dieser Verbindungen mit Fluorsulfonsäure einsetzt.

**13.** Verfahren nach Anspruch 12, dadurch gekennzeichnet, daß man zur Herstellung von $CHCl_2CF_3$ ausgeht von $CCl_2 = CCl_2$, dieses mit der mindestens einfachen der stöchiometrisch zur Fluorwasserstoffanlagerung und zum ChlorFluor-Austausch am Alken notwendigen Menge an Fluorwasserstoff umsetzt, wobei man als Katalysatorgemisch Gemische von Niobpentahalogenid, Tantalpentahalogenid oder Gemische dieser Verbindungen mit Fluorsulfonsäure einsetzt.

**14.** Verfahren nach Anspruch 1, 2 oder 3, dadurch gekennzeichnet, daß man zur Herstellung von $CH_2FCF_3$ ausgeht von $CHF = CF_2$.

**15.** Katalysatorgemisch, umfassend ein Gemisch von Metallhalogenid und einem Sulfonsäurederivat im Mol-Verhältnis von 100:1 bis 1:10, wobei das Metallhalogenid ausgewählt ist aus der Niobpentahalogenid, Tantalpentahalogenid und Molybdänpentahalogenid oder deren Gemische umfassenden Gruppe und das Sulfonsäurederivat ausgewählt wird aus der Fluorsulfonsäure, Perfluorniedrigalkansulfonsäuren mit 1 bis 4 C-Atomen, insbesondere Trifluormethansulfonsäure umfassenden Gruppe.

**16.** Katalysatorgemisch nach Anspruch 15, erhältlich durch Vermischen von Metallhalogenid, Sulfonsäurederivat und Fluorwasserstoff.

**17.** Katalysatorgemisch nach Anspruch 15 oder 16 dadurch gekennzeichnet, daß das Metallhalogenid und das Sulfonsäurederivat im Mol-Verhältnis von 10:1 bis 1:3, insbesondere 2:1 bis 1:2 vorliegen.

**18.** Katalysatorgemisch nach Anspruch 15 bis 17, umfassend ein Gemisch von Niobpentahalogenid, Tantalpentahalogenid oder deren Gemischen als Metallhalogenid und Fluorsulfonsäure, Trifluormethansulfonsäure oder deren Gemischen als Sulfonsäurederivat.

**19.** Katalysatorgemisch nach Anspruch 18, umfassend ein Gemisch von Niobpentahalogenid oder Tantalpentahalogenid und Fluorsulfonsäure.

**20.** Fluorwasserstofflösung zur Anwendung in einem Verfahren gemäß den Ansprüchen 1 bis 14, enthaltend 0,01 bis 99,99 Gew.-% eines Katalysatorgemisches gemäß den Ansprüchen 15 bis 19.

**Claims**

**1.** A method for the preparation of fluorine-containing ethane derivatives of the general formula (I)

$$F_kH_nCl_{3-k-n}C-CZ^1Z^2F \qquad (I),$$

wherein $Z^1$ and $Z^2$ may be identical or different and stand for hydrogen, fluorine, chlorine or bromine, k is 0, 1 or 2 and n is 1, 2 or 3, by reacting a halogenated alkene or halogenated alkane as the starting compound with hydrogen fluoride in the presence of a catalyst system in the liquid phase at a temperature between 0 and 250°C, with the hydrogen fluoride being present in the liquid phase at least in an equimolar quantity, relative to the starting compound, the molar ratio of starting compound to catalyst system being between 10:1 and 1:100, the catalyst system comprising a mixture of metal halide and a sulphonic acid derivative in a molar ratio of metal halide to sulphonic acid derivative of between 100:1 and 1:10, with the metal halide being selected from niobium pentahalide, tantalum pentahalide, molybdenum pentahalide or mixtures thereof and the sulphonic acid derivative being selected from the group comprising fluorosulphonic acid, perfluoro-lower alkane sulphonic acids with 1 to 4 C atoms, in particular trifluoromethane sulphonic acid, and wherein

a) a compound of the general formula (II)

$$F_kH_mCl_{2-k-m}C = CX^1X^2 \qquad (II),$$

wherein $X^1$ and $X^2$ may be identical or different and stand for hydrogen, fluorine, chlorine or bromine, k has the meaning given above and m is 0, 1 or 2, is used as the halogenated alkene, or
b) a compound of the general formula (III)

$$F_kH_nCl_{3-k-n}C-CY^1Y^2Y^3 \qquad (III),$$

wherein k and n have the above meanings, $Y^1$, $Y^2$ may be identical or different and stand for hydrogen, fluorine, chlorine or bromine and $Y^3$ stands for chlorine or bromine, is used as the halogenated alkane.

2. A method according to Claim 1, characterised in that one operates at a temperature of between 50 and 250°C and a pressure of 1 to 100 bar (absolute).

3. A method according to Claim 2, characterised in that for the preparation of fluorine-containing ethane derivatives of the general formula (Ia)

$F_kH_nCl_{3-k-n}C\text{-}CF_3$      (Ia),

wherein k stands for 0, 1 or 2 and n stands for 1, 2 or 3, at a temperature of 50 to 250°C and a pressure of 1 to 100 bar
a) a halogenated alkene of the general formula

$F_kH_mCl_{2-k-m}C = CX^1X^2$      (II),

wherein k and m have the meanings given above, $X^1$ and $X^2$ stand for fluorine, chlorine or bromine, is reacted with hydrogen fluoride, with the hydrogen fluoride being present in the reaction mixture in a quantity which corresponds to the at least single quantity of the quantity stoichiometrically necessary for the hydrogen fluoride addition and for the halogen-fluorine exchange in the alkene or
b) a halogenated alkane of the general formula

$F_kH_nCl_{3-k-n}C\text{-}CY^1Y^2Y^3$      (III),

wherein k and n have the above meanings, $Y^1$ and $Y^2$ are fluorine, chlorine or bromine and $Y^3$ is chlorine or bromine, is reacted with hydrogen fluoride, with the hydrogen fluoride in the reaction mixture being present in a quantity which corresponds to the at least single quantity of the quantity stoichiometrically necessary for the halogen-fluorine exchange in the alkane.

4. A method according to Claim 3, characterised in that for the preparation of $CHCl_2CF_3$
a) $CCl_2 = CCl_2$ is reacted with hydrogen fluoride, with the quantity of hydrogen fluoride in the reaction mixture corresponding to the at least single quantity which is stoichiometrically necessary for the hydrogen fluoride addition and for the chlorine-fluorine exchange on the alkene, or
b) $CHCl_2CCl_3$, $CHCl_2CFCl_2$, $CHCl_2CF_2Cl$ or mixtures thereof are reacted with hydrogen fluoride, with the quantity of hydrogen fluoride in the reaction mixture corresponding to the at least single quantity which is stoichiometrically necessary for the chlorine-fluorine exchange on the alkane.

5. A method according to one of the preceding Claims, characterised in that the starting point is a halogenated alkene.

6. A method according to Claim 4 or 5, characterised in that the starting point for the preparation of $CHCl_2CF_3$ is $CCl_2 = CCl_2$.

7. A method according to Claim 6, characterised in that the molar ratio of $CCl_2 = CCl_2$ and hydrogen fluoride is between 1:3 and 1:100.

8. A method according to Claim 3, characterised in that one operates at a temperature of 80 to 210°C and a pressure of 10 to 40 bar (absolute).

9. A method according to one of the preceding Claims, characterised in that the metal halide and the sulphonic acid derivative are used in a molar ratio of between 10:1 and 1:3.

10. A method according to one of the preceding Claims, characterised in that the molar ratio of starting compound to the catalyst mixture is between 10:1 and 1:10.

14

**11.** A method according to one of the preceding Claims, characterised in that fluorosulphonic acid is used as the sulphonic acid derivative.

**12.** A method according to Claim 11, characterised in that mixtures of niobium pentahalide, tantalum pentahalide or mixtures of these compounds with fluorosulphonic acid are used as the catalyst mixture.

**13.** A method according to Claim 12, characterised in that for the preparation of $CHCl_2CF_3$ the starting point is $CCl_2 = CCl_2$, the latter is reacted with the at least single quantity of hydrogen fluoride stoichiometrically necessary for hydrogen fluoride addition and for the chlorine-fluorine exchange on the alkene, with mixtures of niobium pentahalide, tantalum pentahalide or mixtures of these compounds with fluorosulphonic acid being used as the catalyst mixture.

**14.** A method according to Claim 1, 2 or 3, characterised in that for the preparation of $CH_2FCF_3$ the starting point is $CHF = CF_2$.

**15.** A catalyst mixture, comprising a mixture of metal halide and a sulphonic acid derivative in a molar ratio of 100:1 to 1:10, with the metal halide being selected from the group comprising niobium pentahalide, tantalum pentahalide and molybdenum pentahalide or mixtures thereof and the sulphonic acid derivative being selected from the group comprising fluorosulphonic acid, perfluoro-lower alkane sulphonic acids with 1 to 4 C atoms, in particular trifluoromethane sulphonic acid.

**16.** A catalyst mixture according to Claim 15, obtainable by mixing metal halide, sulphonic acid derivative and hydrogen fluoride.

**17.** A catalyst mixture according to Claim 15 or 16, characterised in that the metal halide and the sulphonic acid derivative are present in a molar ratio of 10:1 to 1:3, in particular 2:1 to 1:2.

**18.** A catalyst mixture according to Claims 15 to 17, comprising a mixture of niobium pentahalide, tantalum pentahalide or mixtures thereof as the metal halide and fluorosulphonic acid, trifluoromethane sulphonic acid or mixtures thereof as the sulphonic acid derivative.

**19.** A catalyst mixture according to Claim 18, comprising a mixture of niobium pentahalide or tantalum pentahalide and fluorosulphonic acid.

**20.** A hydrogen fluoride solution for use in a method according to Claims 1 to 14, containing 0.01 to 99.99% by weight of a catalyst mixture according to Claims 15 to 19.

**Revendications**

**1.** Procédé pour la préparation de dérivés d'éthane contenant du fluor de formule générale (I)

$$F_kH_nCl_{3-k-n}C\text{-}CZ^1Z^2F \qquad (I)$$

dans laquelle $Z^1$ et $Z^2$ peuvent être identiques ou différents et signifient l'hydrogène, le fluor, le chlore ou le brome, k représente 0, 1 ou 2 et n représente 1, 2 ou 3, par réaction d'un alcène halogéné ou d'un alcane halogéné en tant que composé de départ avec de l'acide fluorhydrique en présence d'un système de catalyseur en phase liquide, à une température comprise entre 0 et 250°C, auquel cas l'acide fluorhydrique est présent dans la phase liquide en une quantité au moins équimolaire par rapport au composé de départ, le rapport molaire du composé de départ au système de catalyseur se situe entre 10:1 et 1:100, le système de catalyseur comprend un mélange d'halogénure métallique et d'un dérivé d'acide sulfonique dans le rapport molaire d'halogénure métallique au dérivé d'acide sulfonique compris entre 100:1 et 1:10, l'halogénure métallique étant choisi dans le groupe comprenant un pentahalogénure de niobium, un pentahalogénure de tantale, un pentahalogénure de molybdène ou leurs mélanges et le dérivé d'acide sulfonique étant choisi dans le groupe comprenant l'acide fluorosulfonique, des acides perfluoroalcane(inférieur) sulfoniques avec 1 à 4 atomes de C, notamment l'acide trifluorométhanesulfonique, procédé dans lequel
    a) on utilise en tant qu'alcène halogéné un composé de formule générale (II)

$$F_kH_mCl_{2-k-m}C=CX^1X^2 \qquad (II)$$

dans laquelle $X^1$ et $X^2$ peuvent être identiques ou différents et signifient l'hydrogène, le fluor, le chlore ou le brome, k a la signification indiquée précédemment et m représente 0, 1 ou 2 ; ou
b) on utilise en tant qu'alcane halogéné un composé de formule générale (III)

$$F_kH_nCl_{3-k-n}C\text{-}CY^1Y^2Y^3 \qquad (III)$$

dans laquelle k et n possèdent la signification précédente, $Y^1$, $Y^2$ peuvent être identiques ou différents et signifient l'hydrogène, le fluor, le chlore ou le brome et $Y^3$ signifie le chlore ou le brome.

2. Procédé selon la revendication 1, caractérisé en ce que l'on opère à une température entre 50 et 250°C et sous une pression de 1 à 100 bars (abs.)

3. Procédé selon la revendication 2, caractérisé en ce que, pour la préparation de dérivés d'éthane contenant du fluor de formule générale (Ia)

$$F_kH_nCl_{3-k-n}C\text{-}CF_3 \qquad (Ia)$$

dans laquelle k signifie 0, 1 ou 2 et n signifie 1, 2 ou 3, on fait réagir à une température de 50 à 250°C et sous une pression de 1 à 100 bars
a) un alcène halogéné de formule générale

$$F_kH_mCl_{2-k-m}C=CX^1X^2 \qquad (II)$$

dans laquelle k et m possèdent la signification précédente, $X^1$ et $X^2$ signifient le fluor, le chlore ou le brome, avec de l'acide fluorhydrique, auquel cas l'acide fluorhydrique est présent dans le mélange réactionnel en une quantité qui correspond à au moins la simple quantité stoechiométriquement nécessaire à la fixation par addition de l'acide fluorhydrique et à l'échange halogène-fluor dans le cas de l'alcène ou
b) on fait réagir un alcane halogéné de formule générale

$$F_kH_nCl_{3-k-n}C\text{-}CY^1Y^2Y^3 \qquad (III)$$

dans laquelle k et n possèdent la signification précédente, $Y^1$, $Y^2$ signifient le fluor, le chlore ou le brome et $Y^3$ signifie le chlore ou le brome, avec de l'acide fluorhydrique, auquel cas l'acide fluorhydrique est présent dans le mélange réactionnel en une quantité qui correspond au moins à la simple quantité stoechiométriquement nécessaire à l'échange halogène-fluor dans le cas de l'alcane.

4. Procédé selon la revendication 3, caractérisé en ce que, pour la préparation de $CHCl_2CF_3$,
a) on fait réagir $CCl_2=CCl_2$ avec de l'acide fluorhydrique, la quantité d'acide fluorhydrique dans le mélange réactionnel correspondant à au moins la quantité simple qui est stoechiométriquement nécessaire à la fixation par addition de l'acide fluorhydrique et à l'échange chlore-fluor sur l'alcène, ou
b) on fait réagir $CHCl_2CCl_3$, $CHCl_2CFCl_2$, $CHCl_2CF_2Cl$ ou leurs mélanges avec de l'acide fluorhydrique, la quantité d'acide fluorhydrique dans le mélange réactionnel correspondant à au moins la quantité simple qui est stoechiométriquement nécessaire à l'échange chlore-fluor sur l'alcane.

5. Procédé selon l'une des revendications précédentes, caractérisé en ce que l'on part d'un alcène halogéné.

6. Procédé selon la revendication 4 ou 5, caractérisé en ce que, pour la préparation de $CHCl_2CF_3$, on part de $CCl_2=CCl_2$.

7. Procédé selon la revendication 6, caractérisé en ce que le rapport molaire de $CCl_2=CCl_2$ et d'acide fluorhydrique se situe entre 1:3 et 1:100.

8.  Procédé selon la revendication 3, caractérisé en ce que l'on opère à une température de 80 à 210°C et sous une pression de 10 à 40 bars (abs.).

9.  Procédé selon l'une des revendications précédentes, caractérisé en ce qu'on met en oeuvre l'halogénure métallique et le dérivé d'acide sulfonique dans un rapport molaire entre 10:1 et 1:3.

10. Procédé selon l'une des revendications précédentes, caractérisé en ce que le rapport molaire du composé de départ au mélange de catalyseurs se situe entre 10:1 et 1:10.

11. Procédé selon l'une des revendications précédentes, caractérisé en ce qu'on emploie de l'acide fluorosulfonique comme dérivé d'acide sulfonique.

12. Procédé selon la revendication 11, caractérisé en ce qu'on met en oeuvre en tant que mélange de catalyseurs des mélanges de pentahalogénure de niobium, de pentahalogénure de tantale ou des mélanges de ces composés avec de l'acide fluorosulfonique.

13. Procédé selon la revendication 12, caractérisé en ce que, pour la préparation de $CHCl_2CF_3$, on part de $CCl_2=CCl_2$, fait réagir celui-ci avec au moins la simple quantité d'acide fluorhydrique stoechiométriquement nécessaire à la fixation par addition de l'acide fluorhydrique et à l'échange chlore-fluor sur l'alcène, moyennant quoi on met en oeuvre comme mélange de catalyseurs des mélanges de pentahalogénure de niobium, de pentahalogénure de tantale ou des mélanges de ces composés avec de l'acide fluorosulfonique.

14. Procédé selon la revendication 1, 2 ou 3, caractérisé en ce que, pour la préparation de $CH_2FCF_3$, on part de $CHF=CF_2$.

15. Mélange de catalyseurs comprenant un mélange d'halogénure métallique et d'un dérivé d'acide sulfonique dans le rapport molaire de 100:1 à 1:10, l'halogénure métallique étant choisi dans le groupe comprenant du pentahalogénure de niobium, du pentahalogénure de tantale et du pentahalogénure de molybdène ou leurs mélanges et le dérivé d'acide sulfonique étant choisi dans le groupe comprenant l'acide fluorosulfonique, des acides perfluoroalcane(inférieur)sulfoniques avec 1 à 4 atomes de C, en particulier l'acide trifluorométhanesulfonique.

16. Mélange de catalyseurs selon la revendication 15, pouvant être obtenu par mélange d'un halogénure métallique, d'un dérivé d'acide sulfonique et d'acide fluorhydrique.

17. Mélange de catalyseurs selon la revendication 15 ou 16, caractérisé en ce que l'halogénure métallique et le dérivé d'acide sulfonique sont présents dans le rapport molaire de 10:1 à 1:3, en particulier de 2:1 à 1:2.

18. Mélange de catalyseurs selon les revendications 15 à 17, comprenant un mélange de pentahalogénure de niobium, de pentahalogénure de tantale ou leurs mélanges en tant qu'halogénure métallique et de l'acide fluorosulfonique, de l'acide trifluorométhanesulfonique ou leurs mélanges en tant que dérivé d'acide sulfonique.

19. Mélange de catalyseurs selon la revendication 18, comprenant un mélange de pentahalogénure de niobium ou de pentahalogénure de tantale et d'acide fluorosulfonique.

20. Solution d'acide fluorhydrique pour utilisation dans un procédé selon les revendications 1 à 14, contenant 0,01 à 99,99% en poids d'un mélange de catalyseurs selon les revendications 15 à 19.